# EUROPEAN PATENT SPECIFICATION

(11) **EP 0 988 044 B1**
(45) Date of publication and mention of the grant of the patent: **02.01.2008**
(21) Application number: 98900815.6
(22) Date of filing: 22.01.1998
(51) Int. Cl.: A61K 36/00

(54) **WATER/ETHANOL CENTIPEDA CUNNINGHAMII PLANT EXTRACT**
WASSER/ETHANOL PFLANZENEXTRAKTE AUS CENTIPEDA CUNNINGHAMII
EXTRAIT HYDRO-ETHANOLIQUE DE PLANTES CENTIPEDA CUNNINGHAMII

(30) Priority: 28.01.1997 AU PO480397; 06.11.1997 AU PP022097
(43) Date of publication of application: 29.03.2000
(73) Proprietor: d'Amelio, Frank, Hauppauge, NY 11788 (US); Close, Graeme A., Collingwood, Vic 3066 (AU)
(72) Inventor: CLOSE, Graeme, A., Collingwood, VIC 3066 (AU)
(74) Representative: Hayes, Adrian Chetwynd
(86) International application number: PCT/AU1998/000031
(87) International publication number: WO 1998/032454

(56) References cited:
- AU-A- 3 264 984
- TAYLOR R.S. ET AL.: "Screening of selected medicinal plants of Nepal for antimicrobial activity." J. ETHNOPHARMACOLOGY, vol. 46, 1995, pages 153-159, XP002217216
- SANKAWA, USIO ET AL: "Anti-allergic substances from Chinese medicinal plants" ADV. CHIN. MED. MATER. RES., INT. SYMP. (1985), MEETING DATE 1984, 171-80. EDITOR(S): CHANG, H. M. PUBLISHER: WORLD SCI., SINGAPORE, SINGAPORE. , XP001106027
- CRIBB AB & JW, "Wild Medicine in Australia", COLLINS (SYDNEY), 1988, pages 23-24, 64-65.
- CHEM. PHARM. BULL., (1991), 39(12), WU J. et al., "Biologically Active Constituents of Centipeda Minima: Sesquiterpenes of Potential Anti-Allergy Activity", pages 3272-3275.
- CHEM. PHARM. BULL., (1992), 40(5), IWAKAMI S. et al., "Platelet Activating Factor (PAF) Antagonists Contained in Medicinal Plants: Ligands and Sequiterpenes", pages 1196-1198.
- CHEM. PHARM. BULL., (1985), 33(9), WU J. et al., "Biologically Active Constituents of Centipeda Minima: Isolation of a New Plenolin Ester and the Antiallergy Activity of Sesquiterpene Lactones", pages 4091-4094.
- MUTATION RES., (1988), 204, LEE H. et al., "Antimutagenic Activity of Extracts from Anticancer Drugs in Chinese Medicine", pages 229-234.
- ORAL MICROBIOL. IMMUNOL., (1990), 5, KOKEGUCHI S. et al., "Isolation and Characterization of Lipopolysaccharide from Centipeda Periodontii ATCC 35019", pages 108-112.
- DERWENT ABSTRACT, Accession No. 97-333517; & CN,A,1 106 684 (HUANG J.) 16 August 1995.
- DERWENT ABSTRACT, Accession No. 97-320459; & CN,A,1 105 576 (LIU W) 26 July 1995.
- DERWENT ABSTRACT, Accession No. 97-311152; & CN,A,1 104 506 (UNIVERSITY BEIJING MEDICAL RENMIN HOSPITAL) 5 July 1995.
- DERWENT ABSTRACT, Accession No. 97-154899; & CN,A,1 091 020 (UNIVERSITY BEIJING MEDICAL PEOPLE'S HOSPITAL) 24 August 1994.

## Description

The present invention relates generally to a novel plant composition. More particularly, the present invention provides a composition comprising an extract from the plant *Centipeda cunninghamii* or related *Centipeda* species and an orally or topically acceptable carrier. The *Centipeda* plant extracts of the present invention are useful in a range of cosmetic, therapeutic and prophylactic applications.

Throughout this specification unless the context requires otherwise, the word "comprise", or variations such as "comprises" or "comprising", will be understood to imply the inclusion of a stated integer or group of integers but not the exclusion of any other integer or group of integers.

The plant kingdom has provided an abundant source of many compounds for use in a wide range of cosmetic, therapeutic and prophylactic applications. Indeed, throughout history plants or parts thereof have been used in many ways including the making of oral, topical, and injectable preparations.

*Centipeda cunninghamii* and related plant species have been used by the Aboriginal people of Australia for many generations in the treatment and prevention of many ailments. *C. cunninghamii* is commonly know as Gukwonderuk, koona puturku, sneezeweed, old man weed or scent wood.

*C. cunninghamii* is known to grow primarily in the lower temperate regions of south-eastern Australia. It is commonly found along stream banks and in the back wash of rivers or streams where the water is stagnant.

Traditionally *C. cunninghamii* has been used by the Aboriginal people as a tea, which has either been taken orally or used directly on the skin. Although *C. cunninghamii* has been used by the Aboriginal people there is little known about this plant and the active component(s) responsible for its various properties. Investigations by the present inventors have provided a method for preparing an extract from *C. cunninghamii* and related *Centipeda* species in order to effectively obtain active compounds.

In work leading up to the present invention, the inventors sought to develop compositions comprising an extract from plants in the genus *Centipeda* useful in the effective treatment and prophylaxis of many types of medical problems as well as for cosmetic applications. The inventors have now surprisingly discovered that when an aqueous alcoholic extract of *C. cunninghamii. or* a related *Centipeda* species is combined with a variety of suitable carriers it is useful for topical or oral treatment and prophylaxis of many types of medical conditions as well as for cosmetic applications.

Accordingly, one aspect of the present invention contemplates a composition for the treatment and prophylaxis of medical conditions and/or for cosmetic treatment of a human or other animal, comprising an aqueous alcoholic extract from the plant *Centipeda* Cunninghamii, together with an acceptable carrier and/or diluent, wherein the aqueous alcoholic extract is an extract of plant material with aqueous ethanol in the range of from 10% ethanol /90% water to 95% ethanol 15% water.

The compositions of the present invention have a wide range of utilities in the topical treatment of, for example, acne and pimples, herpes, bed sores, skin irritations, cuts, psoriasis, skin sun damage, eczema and dermatitis. In addition, the compositions are useful in the oral treatment of, for example, nasal irritations and are effective as diuretics. The compositions of the present invention appear to have several properties, such as being anti-allergy, anti-irritant, anti-bacterial and anti-inflammatory. In addition, they are effective as skin proliferators in, for examples, cosmetic applications and skin disease.

The present invention is exemplified herein with respect to humans. However, this is done with the understanding that the present invention extends to all animal including humans, laboratory test animals (e.g. primates, mice, rabbits, hamsters, guinea pigs), livestock animals (e.g. sheep, goats, horses, pigs, cows, donkeys), companion animals (e.g. dogs, cats), captive wild animals (e.g. foxes, kangaroos, dingoes), poultry birds (e.g. chickens, geese, ducks), game birds (e.g. emus, ostriches) and fish and reptiles (eg. fish, lizards, snakes, turtles).

The aqueous alcoholic extract contemplated by the present invention is generally prepared from part (i.e. leaves, flowers, seeds, stems, roots) or all of the *Centipeda cunninghamii* plant or other related species of *Centipeda.* The aqueous alcoholic extract can be prepared by utilizing an adequate extraction method (eg. by maceration, percolation, infusion, decoction) consisting of, but not limited to, various alcoholic solvents such as aqueous ethanolic solvents. A preferred extract is obtained by extraction of dried plant material with aqueous ethanol, comprising 30% ethanol/70% water or a range of strengths.

Topical compositions of the present invention comprise an aqueous alcoholic extract together with a suitable carrier for topical administration. For example, the extract may be combined with one or more of the following carriers:
emulsifiers (eg. beeswax, stearic acid), skin protectants (eg. dimethicone), skin conditioners (eg. olive oil, avocado oil, cetyl stearyl alcohol), humectants (eg. glycerin), preservatives (eg. methyl hydroxybenzoate), solvents (eg. water), herbal actives (eg. aloe vera), essential oils (eg. sandalwood oil, lavender oil), suspension agents (eg. fractionated coconut oil), anionic emulsifying agents (eg. cocoamidopropyl betaine), astringents (eg. zinc phenosulfonate), antiseptics (eg. zinc chloride), antibacterials (eg. triclosan), healing agents (eg. allantoin), germicides (eg. tea tree oil), anti-inflammatories (eg. pineapple extract), skin fresheners (eg. lemon extract), wetting agents (eg. cocoamidopropyl betaine), antioxidants (eg. lecithin), skin lubricants (eg. jojoba oil), emollients (eg. honey), absorbing agents (eg. purified talc), preservatives (eg. methyl hydroxybenzoate) and/or skin/hair conditioners (eg. olive oil).

Oral compositions of the present invention comprise an aqueous alcoholic extract together with a suitable carrier for oral administration. For example, the extract may be combined with one or more of the following carriers:
abrasives (eg. calcium carbonate), solvents (eg. water), humectants (eg. glycerine), detergents (eg. sodium lauryl sulphate), binders (eg. cellulose gum), herbal actives (eg. aloe vera), essential oils (eg. eucalyptus oil, peppermint oil), deodorizing agents (eg. chlorophyll) and/or a suspension agents (eg. hydrogenated castor oil).

Another aspect of the present invention provides a method of treatment or prophylaxis of medical conditions and/or a method of cosmetic treatment of a human or other animal, comprising administration of a composition comprising an aqueous alcoholic extract from a plant of the genus *Centipeda,* combined with an acceptable carrier and/or diluent.

Medical conditions contemplated by the present invention comprise but are not limited to rashes and allergic reactions, inflammations, bacterial infections and gastrointestinal problems generally.

Cosmetic treatments contemplated by the present invention comprise but are not limited to skin and/or hair treatments and/or oral treatments, such as, deodorants, facial scrubs, shaving creams and gels, eye/face creams and gels, make-up removers, toners, cleansers, shampoos, conditioners and breath fresheners.

A variety of administration routines are available. The particular mode selected will depend, of course, upon the particular condition being treated and the dosage required for therapeutic, prophylactic or cosmetic efficacy. The methods of this invention, generally speaking, may be practised using any mode of administration that is topically or orally acceptable, meaning any mode that produces effective levels of the active component of the invention without causing clinically unacceptable adverse effects. Such modes of administration include oral (eg. tablets, mouth washes, mouth gels, mouth lotions, gargle solutions, toothpastes, emmenagogues) and topical (eg. creams, paints, sprays, pastes, liniments, lotions, ointments).

The compositions of this invention may conveniently be presented in unit dosage form and may be prepared by any of the methods well known in the art of pharmacy and cosmetics. Such methods include the step of bringing the active component into association with a carrier which constitutes one or more accessory ingredients.

The formulation of such therapeutic, prophylactic and cosmetic compositions is well known to persons skilled in these fields. Suitable pharmaceutically acceptable carriers and/or diluents include any and all conventional solvents, dispersion media, fillers, solid carriers, aqueous solutions, coatings, antibacterial and antifungal agents, isotonic and absorption delaying agents, and the like. The use of such media and agents for pharmaceutically active substances is well known in the art, and it is described, by way of example in Remington's Pharmaceutical Sciences, 18th Edition, Mack Publishing Company, Pennsylvania, USA. Except insofar as any conventional media or agent is incompatible with the active component, use thereof in the compositions of the present invention is contemplated. Supplementary active ingredients can also be incorporated into the compositions.

Accordingly, a further aspect of the present invention contemplates a method of oral treatment or prophylaxis of medical conditions of a human or other animal, comprising oral administration of an aqueous alcoholic extract from a plant of the genus *Centipeda,* combined with an orally suitable carrier.

Compositions of the present invention suitable for oral administration may be presented as discrete units such as ingestible tablets, buccal tablets, capsules, lozenges, cachets, wafers and the like, each containing a predetermined amount of the active component, in liposomes or as a suspension in an aqueous liquid or non-liquid such as a syrup, an elixir, or an emulsion.

The oral treatment contemplated by the present invention includes, for example, the swallowing or ingestion of tablets, capsules, lozenges, cachets, wafers, solutions, syrups, elixirs or emulsions. In addition, oral treatment includes compositions applied within the mouth cavity for a local effect, for example, as mouth lotions, mouth gels or gargle solutions.

Accordingly, a further aspect of the present invention contemplates a method of topical treatment or prophylaxis of medical conditions or a topical cosmetic treatment of a human or other animal, comprising topical administration of an aqueous alcoholic extract from a plant of the genus *Centipeda,* combined with an topically suitable carrier.

Compositions of the present invention suitable for topical administration may be presented as a liquid or semi-liquid preparation intended for application to the skin, hair, nails or nasal passages, for example, as follows:
a cream a homogeneous viscous or sem-solid emulsion, a gel (eg. semi-solid preparation), a liniment (ie. a liquid preparation), a lotion (ie. a semi-liquid preparation), an ointment (ie. a semi-solid preparation), a paint (ie. a liquid preparation that is brushed onto the skin), a paste (ie. a semi-solid preparation for external application), a solution (ie. a liquid preparation), a spray (ie. a liquid preparation dispersed with a spraying device), a stick (ie. a solid preparation) or a suspension (ie. a liquid preparation).

Throughout this specification unless the context requires otherwise, the terms "aqueous alcoholic extract", *"Centipeda* plant extract" and "plant extract" are intended to have the same meaning.

Further features of the present invention are more fully described in the following Example(s). It is to be understood, however, that this detailed description is included solely for the purposes of exemplifying the present invention, and should not be understood in any way as a restriction on the broad description of the invention as set out above.

### Example 1

### Procedure for preparation of the aqueous alcohol extract from C. cunninghamii

- PROCEDURES:: Cold maceration process using aqueous/alcohol in a range of strengths i.e. 10% Ethanol:Water to 95% Ethanol:Water.
- SOLVENTS USED:: Ethanol all types i.e. 100SG 95SG ETC
Denatured ethanol all types i.e. F1-F2 etc
Water all types, i.e. purified, distilled, potable, etc.
- PLANT PARTS USED:: Whole plant, leaves, flowers, seeds, stems, roots.
- RATIO:: Dried plant = 6-1 to 7-1 of fresh plant depending on dryness.
- TIME;: Maceration time = 6 weeks for 30% Ethanol:Water, but can vary with different levels of solvent strengths
- PROTOCOL:: 20-23.5kg of dried plant whole or chopped into small pieces are placed into a vessel i.e. stainless steel, poly etc with a range of Ethanol:Water strengths ranging from 10% Ethanol Water to 95% Ethanol:Water and left to macerate for a period of approximately b weeks, at the completion the material is pressed and the liquid is filtered.

The recovered liquid can vary depending on Ethanol:Water strength.

### Example 2

### BABY NAPPY CHANGE CREAM: Barrier Cream

The *Centipeda* plant extract of Example 1 was mixed with the ingredients listed below. This composition can be applied topically and is suitable for use as a barrier cream in particular as a baby nappy change cream.

| **INGREDIENTS** | **%** | **FUNCTION** |
|---|---|---|
| BEESWAX | 0.6 | EMULSIFIER |
| CETYL STEARYL ALCOHOL | 7.5 | SKIN CONDITIONER |
| LANOLIN WAX | 1.0 | EMULSIFIER |
| CETOMACROGOL 1000 | 1.6 | EMULSIFIER |
| DIMETHICONE | 2.0 | SKIN PROTECTANT |
| OLIVE OIL | 7.5 | SKIN CONDITIONER |
| GLYCERIN | 5.0 | HUMECTANT |
| METHYL HYDROXYBENZOATE | 0.3 | PRESERVATIVE |
| PURIFIED WATER | 54.5 | SOLVENT |
| ALOE VERA | 6.0 | HERBAL ACTIVE |
| WATTLE EXTRACT 1-1 | 2.0 | HERBAL ACTIVE |
| *CENTIPEDA* PLANT EXTRACT 1-1 | 6.0 | HERBAL ACTIVE |
| CHAMOMILE EXTRACT 1-1 | 1.0 | HERBAL ACTIVE |
| LAVENDER EXTRACT 1-1 | 2.2 | HERBAL ACTIVE |
| SANDALWOOD OIL | 0.3 | ESSENTIAL OIL |
| LAVENDER OIL | 0.6 | ESSENTIAL OIL |
| LAURYL ALCOHOL | 0.5 | EMULSIFIER |
| FRACTIONATED COCONUT OIL | 0.4 | SUSPENSION AGENT |
| CAJUPUT OIL | 1.0 | ESSENTIAL OIL |
| TOTAL | 100.0 | |

### Example 3

### BABY SOOTHING SKIN CREAM

The *Centipeda* plant extract of Example I was mixed with the ingredients listed below. This composition can be applied topically and is suitable for use as a baby sooting skin cream.

| **INGREDIENTS** | **%** | **FUNCTION** |
|---|---|---|
| CETYL STEARYL ALCOHOL | 9.0 | SKIN CONDITIONER |
| CETOMACROGOL 1000 | 1.6 | EMULSIFIER |
| OLIVE OIL | 7.5 | SKIN CONDITIONER |
| GLYCERIN | 4.5 | HUMECTANT |
| METHYL HYDROXYBENZOATE | 0.3 | PRESERVATIVE |
| PURIFIED WATER | 53.1 | SOLVENT |
| ALOE VERA | 7.2 | HERBAL ACTIVE |
| *CENTIPEDA* PLANT EXTRACT 1-1 | 8.0 | HERBAL ACTIVE |
| LAVENDER EXTRACT 1-1 | 1.6 | HERBAL ACTIVE |
| CALENDULA EXTRACT 1-1 | 2.0 | HERBAL ACTIVE |
| WATTLE EXTRACT 1-1 | 3.0 | HERBAL ACTIVE |
| LAURYL ALCOHOL | 0.5 | EMULSIFIER |
| FRACTIONATED COCONUT OIL | 0.4 | SUSPENSION AGENT |
| LEMON OIL | 0.4 | ESSENTIAL OIL |
| LAVENDER OIL | 0.2 | ESSENTIAL OIL |
| CAJUPUT OIL | 0.3 | ESSENTIAL OIL |
| EUCALYPTUS OIL | 0.2 | ESSENTIAL OIL |
| PINE NEEDLE OIL | 0.1 | ESSENTIAL OIL |
| PEPPERMINT OIL | 0.1 | ESSENTIAL OIL |
| TOTAL | 100.0 | |

### Example 4

### BABY MOISTURISING LOTION

The *Centipeda* plant extract of Example I was mixed with the ingredients listed below. This composition can be applied topically and is suitable for use as a baby moisturising lotion.

| **INGREDIENTS** | **%** | **FUNCTION** |
|---|---|---|
| LANOLIN ALCOHOL | 1.8 | EMULSIFIER |
| STEARIC ACID | 3.2 | EMULSIFIER |
| CETYL STEARYL ALCOHOL | 1.2 | SKIN CONDITIONER |
| METHYL HYDROXYBENZOATE | 0.2 | PRESERVATIVE |
| OLIVE OIL | 7.6 | SKIN CONDITIONER |
| GLYCERIN | 4.0 | HUMECTANT |
| PURIFIED WATER | 62.0 | SOLVENT |
| LAURYL ALCOHOL | 0.5 | EMULSIFIER |
| FRACTIONATED COCONUT OIL | 0.4 | SUSPENSION AGENT |
| ALOE VERA | 8.0 | HERBAL ACTIVE |
| *CENTIPEDA* PLANT EXTRACT 1-1 | 3.0 | HERBAL ACTIVE |
| AVOCADO OIL | 1.4 | SKIN CONDITIONER |
| WHEATGERM OIL | 1.0 | SKIN CONDITIONER |
| MACADAMIA OIL | 1.0 | SKIN CONDITIONER |
| APPLE OIL | 0.4 | ESSENTIAL OIL |
| CHAMOMILE EXTRACT 1-1 | 2.0 | HERBAL ACTIVE |
| CALENDULA EXTRACT 1-1 | 2.0 | HERBAL ACTIVE |
| ORANGE OIL | 0.3 | ESSENTIAL OIL |
| TOTAL | 100.0 | |

### Example 5

### BABY WASH

The *Centipeda* plant extract of Example 1 was mixed with the ingredients listed below. This composition can be applied topically and is suitable for use as a baby wash solution.

| **INGREDIENTS** | | **% FUNCTION** |
|---|---|---|
| PURIFIED WATER | 50.0 | SOLVENT |
| SODIUM LAURYL ETHER SULPHATE | 12.5 | ANIONIC EMULSIFYING AGENT |
| COCOAMIDOPROPYL BETAINE | 8.0 | ANTISTATIC AGENT |
| FRACTIONATED COCONUT OIL | 6.5 | SUSPENSION AGENT |
| ALOE VERA JUICE | 10.0 | HERBAL ACTIVE |
| GLYCERIN | 5.0 | HUMECTANT |
| VIRGIN OLIVE OIL | 2.0 | SKIN CONDITIONER |
| *CENTIPEDA* PLANT EXTRACT 1-1 | 2.5 | HERBAL ACTIVE |
| CHAMOMILE EXTRACT 1-1 | 2.5 | HERBAL ACTIVE |
| APPLE OIL | 0.7 | ESSENTIAL OIL |
| METHYL HYDROXYBENZOATE | 0.2 | PRESERVATIVE |
| PROPYL HYDROXYBENZOATE | 0.1 | PRESERVATIVE |
| TOTAL | 100.0 | |

### Example 6

### HERBAL & MINERAL TOOTHPASTE

The plant extract of Example 1 was mixed with the ingredients listed below. This composition is suitable for use as a toothpaste.

| **INGREDIENTS** | **%** | **FUNCTION** |
|---|---|---|
| CALCIUM CARBONATE | 48.0 | ABRASIVE |
| PURIFIED WATER | 21.82 | SOLVENT |
| GLYCERIN | 22.0 | HUMECTANT |
| SILICA | 1.6 | ABRASIVE |
| SODIUM LAURYL SULPHATE | 2.0 | DETERGENT |
| CELLULOSE GUM | 0.77 | BINDER |
| *CENTIPEDA* PLANT EXTRACT | 2.1 | HERBAL ACTIVE |
| EUCALYPTUS OIL | 0.2 | ESSENTIAL OIL |
| PEPPERMINT OIL | 0.3 | ESSENTIAL OIL |
| CHLOROPHYLL | 0.01 | DEODORIZING AGENT |
| HYDROGENATED CASTOR OIL | 1.2 | SUSPENSION AGENT |
| TOTAL | 100.0 | |

### Example 7

### DEODORANT - ALUMINIDM FREE

### Antiperspirant

The *Centipeda* plant extract of Example 1 was mixed with the ingredients listed below. This composition is suitable for use as an antiperspirant.

| **INGREDIENTS** | **%** | **FUNCTION** |
|---|---|---|
| PURIFIED WATER | 20.0 | SOLVENT |
| ETHANOL | 70.0 | SOLVENT |
| ZINC PHENOSULFONATE | 2.0 | ASTRINGENT |
| ZINC CHLORIDE | 2.0 | ANTISEPTIC |
| TRICLOSAN | 0.2 | ANTIBACTERIAL |
| ALLANTOIN | 0.2 | HEALING AGENT |
| EUCALYPTUS OIL | 0.18 | ANTISEPTIC |
| TEA TREE OIL | 0.2 | GERMICIDE |
| PINE NEEDLE OIL | 0.2 | DEODORANT |
| PINEAPPLE EXTRACT 1-1 | 1.0 | ANTI-INFLAMMATORY |
| LEMON EXTRACT 1-1 | 1.0 | SKIN FRESHENER |
| CHLOROPHYLL | 0.02 | DEODORIZING AGENT |
| *CENTIPEDA* PLANT EXTRACT 1-1 | 3.0 | HERBAL ACTIVE |
| TOTAL | 100.0 | |

### Example 8

### MEN'S FACIAL SCRUB

The *Centipeda* plant extract of Example 1 was mixed with the ingredients listed below. This composition can be applied topically and is suitable for use as a facial scrub.

| **INGREDIENTS** | **%** | **FUNCTION** |
|---|---|---|
| OLIVE OIL | 2.0 | SKIN CONDITIONER |
| LANOLIN ALCOHOL | 1.4 | EMULSIFIER |
| GRAPESEED OIL | 4.2 | SKIN CONDITIONER |
| STEARIC ACID | 3.7 | EMULSIFIER |
| CETYL STEARYL ALCOHOL | 1.9 | SKIN CONDITIONER |
| METHYL HYDROXYBENZOATE | 0.2 | PRESERVATIVE |
| CITRIC ACID | 0.5 | PRESERVATIVE |
| FRACTIONATED COCONUT OIL | 9.5 | SUSPENSION AGENT |
| COCOAMIDOPROPYL BETAINE | 4.5 | WETTING AGENT |
| LECITHIN | 1.2 | ANTIOXIDANT |
| ALOE VERA | 10.0 | HERBAL ACTIVE |
| *CENTIPEDA* PLANT EXTRACT 1-1 | 9.3 | HERBAL ACTIVE |
| ALMOND MEAL | 3.0 | HERBAL ACTIVE |
| PURIFIED WATER | 48.0 | SOLVENT |
| ORANGE OIL | 0.4 | ESSENTIAL OIL |
| GRAPEFRUIT OIL | 0.2 | ESSENTIAL OIL |
| TOTAL | 100.0 | |

### Example 9

### AFTER SHAVE HEALER

The *Centipeda* plant extract of Example 1 was mixed with the ingredients listed below. This composition can be applied topically and is suitable for use an aftershave.

| **INGREDIENTS** | **%** | **FUNCTION** |
|---|---|---|
| ALOE VERA | 20.0 | HERBAL ACTIVE |
| *CENTIPEDA* PLANT EXTRACT 1-1 | 15.0 | HERBAL ACTIVE |
| GLYCERIN | 5.0 | HUMECTANT |
| WITCH HAZEL EXTRACT 1-1 | 5.0 | HERBAL ACTIVE |
| APPLE CIDER VINEGAR | 1.2 | SOLVENT |
| METHYL HYDROXYBENZOATE | 0.2 | PRESERVATIVE |
| FRACTIONED COCONUT OIL | 0.4 | SUSPENSION AGENT |
| LAURYL ALCOHOL | 0.5 | EMULSIFIER |
| PURIFIED WATER | 52.3 | SOLVENT |
| LEMON OIL | 0.4 | ESSENTIAL OIL |
| TOTAL | 100.0 | |

### Example 10

### FOAMING SHAVE GEL

The *Centipeda* plant extract of Example 1 was mixed with the ingredients listed below. This composition can be applied topically and is suitable for use as a foaming shave gel.

| **INGREDIENTS** | **%** | **FUNCTION** |
|---|---|---|
| FRACTIONATED COCONUTOIL | 10.0 | SUSPENSION AGENT |
| COCOAMIDOPROPYL BETAINE | 12.0 | ANTISTATIC AGENT |
| PURIFIED WATER | 37.6 | SOLVENT |
| LAURYL ALCOHOL | 0.7 | EMULSIFIER |
| OLIVE OIL | 4.0 | SKIN CONDITION |
| GLYCERIN | 10.0 | HUMECTANT |
| ALOE VERA | 15.0 | HERBAL ACTIVE |
| *CENTIPEDA* PLANT EXTRACT 1-1 | 10.0 | HERBAL ACTIVE |
| MENTHOL CRYSTALS | 0.1 | SKIN FRESHENER |
| LEMON OIL | 0.4 | ESSENTIAL OIL |
| TEA TREE OIL | 0.2 | ANTISEPTIC |
| TOTAL | 100.0 | |

### Example 11

### MOISTURISER

### For Acne Prone Skin

The *Centipeda* plant extract of Example 1 was mixed with the ingredients listed below. This composition can be applied topically and is suitable for use as a moisturiser.

| **INGREDIENTS** | **%** | **FUNCTION** |
|---|---|---|
| OLIVE OIL | 2.25 | SKIN CONDITIONER |
| LANOLIN ALCOHOLS | 1.75 | EMULSIFIER |
| STEARIC ACID | 3.3 | EMULSIFIER |
| CETYL STEARYL ALCOHOL | 1.1 | SKIN CONDITIONER |
| METHYL HYDROXYBENZOATE | 0.3 | PRESERVATIVE |
| GLYCERIN | 5.0 | HUMECTANT |
| PURIFIED WATER | 49.7 | SOLVENT |
| ALOE VERA 1-1 | 25.0 | HERBAL ACTIVE |
| *CENTIPEDA* PLANT EXTRACT 1-1 | 10.0 | HERBAL ACTIVE |
| TEA TREE OIL | 0.5 | ESSENTIAL OIL |
| SANDALWOOD OIL | 0.01 | ESSENTIAL OIL |
| GERANIUM OIL | 0.09 | ESSENTIAL OIL |
| LAVENDER OIL | 0.1 | ESSENTIAL OIL |
| LAURYL ALCOHOL | 0.5 | EMULSIFIER |
| FRACTIONED COCONUT OIL | 0.4 | SUSPENSION AGENT |
| TOTAL | 100.0 | |

### Example 12

### ACNE GEL

The *Centipeda* plant extract of Example 1 was mixed with the ingredients listed below. This composition can be applied topically and is suitable for use as acne gel.

| **INGREDIENTS** | **%** | **FUNCTION** |
|---|---|---|
| ALOE VERA | 25.0 | HERBAL ACTIVE |
| *CENTIPEDA* PLANT EXTRACT 1-1 | 20.0 | HERBAL ACTIVE |
| METHYL HYDROXYBENZOATE | 0.2 | PRESERVATIVE |
| PURIFIED WATER | 45.0 | SOLVENT |
| FRACTIONATED COCONUT OIL | 0.7 | SUSPENSION AGENT |
| LAURYL ALCOHOL | 0.8 | EMULSIFIER |
| TEA TREE OIL | 0.7 | ESSENTIAL OIL |
| CAJUPUT OIL | 0.5 | ESSENTIAL OIL |
| SANDALWOOD OIL | 0.2 | ESSENTIAL OIL |
| CALENDULA OIL | 2.5 | SKIN CONDITIONER |
| LAVENDER OIL | 0.6 | ESSENTIAL OIL |
| CALENDULA EXTRACT 1-1 | 2.5 | HERBAL ACTIVE |
| GOLDEN SEAL EXTRACT 1-1 | 1.3 | HERBAL ACTIVE |
| TOTAL | 100.0 | |

### Example 13

### ANTI BACTERIAL FACE WASH

The *Centipeda* plant extract of Example 1 was mixed with the ingredients listed below. This composition can be applied topically and is suitable for use as a face wash in particular as an antibacterial wash.

| **INGREDIENTS** | **%** | **FUNCTION** |
|---|---|---|
| FRACTIONED COCONUT OIL | 10.0 | SUSPENSION AGENT |
| COC-BETAINE | 9.0 | ANTISTATIC AGENT |
| GLYCERIN | 10.0 | HUMECTANT |
| SODIUM LAURYL ETHER SULPHATE | 7.0 | WETTING AGENT |
| CITRIC ACID | 0.5 | PRESERVATIVE |
| METHYL HYDROXYBENZOATE | 0.1 | PRESERVATIVE |
| ALOE VERA | 10.0 | HERBAL ACTIVE |
| TEA TREE OIL | 0.1 | ESSENTIAL OIL |
| THYME OIL | 0.1 | ESSENTIAL OIL |
| THYME EXTRACT 1-1 | 2.5 | HERBAL ACTIVE |
| *CENTIPEDA* PLANT EXTRACT 1-1 | 10.0 | HERBAL ACTIVE |
| CALENDULA EXTRACT 1-1 | 5.0 | HERBAL ACTIVE |
| CAJUPUT OIL | 0.2 | ESSENTIAL OIL |
| PURIFIED WATER | 35.5 | SOLVENT |
| TOTAL | 100.0 | |

### Example 14

### REVIVE SHOWER GEL

The *Centipeda* plant extract of Example 1 was mixed with the ingredients listed below. This composition is suitable for use as a shower gel.

| **INGREDIENTS** | **%** | **FUNCTION** |
|---|---|---|
| PURIFIED WATER | 32.8 | SOLVENT |
| SODIUM LAURYL ETHER SULPHATE | 15.0 | EMULSIFYING AGENT |
| COCAMIDOPROPYL BETAINE | 12.0 | WETTING AGENT |
| FRACTIONED COCONUT OIL | 9.0 | SUSPENSION AGENT |
| ALOE VERA JUICE | 10.0 | HERBAL ACTIVE |
| GLYCERIN | 8.0 | HUMECTANT |
| OLIVE OIL | 3.4 | SKIN CONDITIONER |
| AVOCADO OIL | 1.5 | SKIN CONDITIONER |
| WHEATGERM OIL | 1.2 | SKIN CONDITIONER |
| LEMON GRASS OIL | 0.4 | ESSENTIAL OIL |
| PATCHOULI OIL | 0.2 | ESSENTIAL OIL |
| ROSEMARY OIL | 0.2 | ESSENTIAL OIL |
| CITRIC ACID | 1.3 | PRESERVATIVE |
| *CENTIPEDA* PLANT EXTRACT 1-1 | 5.0 | HERBAL ACTIVE |
| TOTAL | 100.0 | |

### Example 15

### KALMING CREAM

The *Centipeda* plant extract of Example 1 was mixed with the ingredients listed below. This composition can be applied topically and is suitable for use as a kalming cream.

| **INGREDIENTS** | **%** | **FUNCTION** |
|---|---|---|
| CETYL STEARYL ALCOHOL | 8.5 | SKIN CONDITIONER |
| CETOMACROGOL 1000 | 1.6 | EMULSIFIER |
| OLIVE OIL | 4.3 | SKIN CONDITIONER |
| GRAPESEED OIL | 3.7 | SKIN CONDITIONER |
| GLYCERIN | 3.0 | HUMECTANT |
| METHYL HYDROXYBENZOATE | 0.2 | PRESERVATIVE |
| *CENTIPEDA* PLANT EXTRACT 1-1 | 6.0 | HERBAL ACTIVE |
| PANTOTHENIC ACID | 0.3 | ACTIVE |
| MACADAMIA OIL | 2.1 | SKIN CONDITIONER |
| ROSEMARY OIL | 0.4 | ESSENTIAL OIL |
| GERANIUM OIL | 0.5 | ESSENTIAL OIL |
| PEPPERMINT OIL | 0.3 | ESSENTIAL OIL |
| BASIL OIL | 0.1 | ESSENTIAL OIL |
| EVENING PRIMROSE OIL | 2.1 | SKIN CONDITIONER |
| CITRIC ACID | 0.5 | PRESERVATIVE |
| PURIFIED WATER | 45.5 | SOLVENT |
| ALOE VERA | 20.0 | HERBAL ACTIVE |
| FRACTIONED COCONUT OIL | 0.4 | SUSPENSION AGENT |
| LAURYL ALCOHOL | 0.5 | EMULSIFIER |
| TOTAL | 100.0 | |

### Example 16

### SOOTHING MASSAGE OIL RUB

The *Centipeda* plant extract of Example 1 was mixed with the ingredients listed below. This composition can be applied topically and is suitable for use as a massage oil rub.

| **INGREDIENTS** | **%** | **FUNCTION** |
|---|---|---|
| OLIVE OIL | 30.0 | SKIN CONDITIONER |
| GRAPESEED OIL | 39.3 | SKIN CONDITIONER |
| AVOCADO OIL | 5,0 | SKIN CONDITIONER |
| ALMOND OIL | 3.0 | SKIN CONDITIONER |
| MACADAMIA OIL | 6.0 | SKIN CONDITIONER |
| LAVENDER OIL | 0.4 | ESSENTIAL OIL |
| JUNIPER OIL | 0.2 | ESSENTIAL OIL |
| ROSEMARY OIL | 0.2 | ESSENTIAL OIL |
| ORANGE OIL | 0.8 | ESSENTIAL OIL |
| JOJOBA OIL | 4.0 | SKIN LUBRICANT |
| CUAIACWOOD OIL | 0.4 | ESSENTIAL OIL |
| ETHANOL | 4.0 | SOLVENT |
| WHEATGERM OIL | 1.7 | SKIN CONDITIONER |
| *CENTIPEDA* PLANT EXTRACT 1-1 | 5.0 | HERBAL ACTIVE |
| TOTAL | 100.0 | |

### Example 17

### RELAXING BATH OIL-FOAMING

The *Centipeda* plant extract of Example 1 was mixed with the ingredients listed below. This composition can be applied topically and is suitable for use as a foaming bath oil.

| **INGREDIENTS** | **%** | **FUNCTION** |
|---|---|---|
| GLYCERIN | 20.0 | HUMECTANT |
| PURIFIED WATER | 18.3 | SOLVENT |
| ALOE VERA JUICE | 15.0 | HERBAL ACTIVE |
| SODIUM LAURYL ETHER SULPHATE | 15.0 | WETTING AGENT |
| FRACTIONED COCONUT OIL | 8.0 | SUSPENSION AGENT |
| OLIVE OIL | 5.0 | SKIN CONDITIONER |
| METHYL HYDROXYBENZOATE | 0.2 | PRESERVATIVE |
| *CENTIPEDA* PLANT EXTRACT 1-1 | 10.0 | HERBAL ACTIVE |
| CHAMOMILE EXTRACT | 5.2 | HERBAL ACTIVE |
| SANDALWOOD OIL | 0.5 | ESSENTIAL OIL |
| YLANG YLANG OIL | 0.8 | ESSENTIAL OIL |
| ROSE OIL | 0.3 | ESSENTIAL OIL |
| GERANIUM OIL | 0.4 | ESSENTIAL OIL |
| LAVENDER OIL | 0.8 | ESSENTIAL OIL |
| ORANGE OIL | 0.5 | ESSENTIAL OIL |
| TOTAL | 100.0 | |

### Example 18

### HEALING CREAM

The *Centipeda* plant extract of Example 1 was mixed with the ingredients listed below. This composition can be applied topically as a healing cream.

| **INGREDIENTS** | **%** | **FUNCTION** |
|---|---|---|
| OLIVE OIL | 7.5 | SKIN CONDITIONER |
| CETYL STEARYL ALCOHOL | 10.0 | SKIN CONDITIONER |
| CETOMACROHOL 1000 | 1.5 | EMULSIFIER |
| METHYL HYDROXYBENZOATE | 0.3 | PRESERVATIVE |
| PROPYL HYDROXYBENZOATE | 0.1 | PRESERVATIVE |
| GLYCERIN | 5.6 | HUMECTANT |
| PURIFIED WATER | 55.0 | SOLVENT |
| ALOE VERA | 3.0 | HERBAL ACTIVE |
| *CENTIPEDA* PLANT EXTRACT 3-1 | 7.0 | HERBAL ACTIVE |
| WATTLE EXTRACT 3-1 | 2.0 | HERBAL ACTIVE |
| CAJUPUT OIL | 0.4 | ESSENTIAL OIL |
| TEA TREE OIL | 1.0 | ESSENTIAL OIL |
| LAVENDER OIL | 0.6 | ESSENTIAL OIL |
| SANDALWOOD OIL | 1.0 | ESSENTIAL OIL |
| CALENDULA EXTRACT 3-1 | 2.1 | HERBAL ACTIVE |
| GOLDEN SEAL EXTRACT 1-1 | 2.0 | HERBAL ACTIVE |
| LAURYL ALCOHOL | 0.5 | EMULSIFIER |
| FRACTIONATED COCONUT OIL | 0.4 | SUSPENSION AGENT |
| TOTAL | 100.0 | |

### Example 19

### BLACK HEAD REMOVER

The *Centipeda* plant extract of Example 1 was mixed with the ingredients listed below. This composition can be applied topically and is suitable for use as a black head remover.

| **INGREDIENTS** | **%** | **FUNCTION** |
|---|---|---|
| PURIFIED WATER | 33.1 | SOLVENT |
| ALOE VERA | 15.0 | HERBAL ACTIVE |
| ETHANOL | 25.0 | SOLVENT |
| HYDROGENATED CASTER OIL | 0.9 | SUSPENSION AGENT |
| *CENTIPEDA* PLANT EXTRACT | 15.0 | HERBAL ACTIVE |
| CHICKWEED EXTRACT 1-1 | 1.6 | HERBAL ACTIVE |
| CHAMOMILE EXTRACT 1-1 | 2.9 | HERBAL ACTIVE |
| DANDELION EXTRACT 1-1 | 1.7 | HERBAL ACTIVE |
| PARSLEY OIL | 0.2 | ESSENTIAL OIL |
| LEMON GRASS OIL | 0.4 | ESSENTIAL OIL |
| YARROW EXTRACT | 2.3 | HERBAL ACTIVE |
| HONEY | 1.1 | EMOLLIENT |
| APPLE CIDER VINEGAR | 0.8 | SOLVENT |
| TOTAL | 100.0 | |

### Example 20

### UNDER EYE CARE GEL

The *Centipeda* plant extract of Example 1 was mixed with the ingredients listed below. This composition can be applied topically and is suitable for use as a cosmetic.

| **INGREDIENTS** | **%** | **FUNCTION** |
|---|---|---|
| ANGELICA EXTRACT 1-1 | 5.0 | HERBAL ACTIVE |
| ALOE VERA JUICE | 25.0 | HERBAL ACTIVE |
| CHAMOMILE EXTRACT 1-1 | 2.0 | HERBAL ACTIVE |
| FRACTIONATED COCONUT OIL | 0.7 | SUSPENSION AGENT |
| LAURYL ALCOHOL | 0.8 | EMULSIFIER |
| METHYL HYDROXYBENZOATE | 0.2 | PRESERVATIVE |
| AVOCADO OIL | 1.5 | SKIN CONDITIONER |
| OLIVE OIL | 3.8 | SKIN CONDITIONER |
| *CENTIPEDA* PLANT EXTRACT 1-1 | 12.0 | HERBAL ACTIVE |
| PURIFIED WATER | 49.0 | SOLVENT |
| TOTAL | 100.0 | |

### Example 21

### EYE SURROUND BALM

The *Centipeda* plant extract of Example 1 was mixed with the ingredients listed below. This composition can be applied topically and is suitable for use as a balm.

| **INGREDIENTS** | **%** | **FUNCTION** |
|---|---|---|
| CETYL STEARYL ALCOHOL | 6.5 | SKIN CONDITIONER |
| CETOMACROGOL 1000 | 1.0 | EMULSIFIER |
| LANOLIN WAX | 0.8 | EMULSIFIER |
| BEESWAX | 0.6 | EMULSIFIER |
| GLYCERIN | 8.0 | HUMECTANT |
| METHYL HYDROXYBENZOATE | 0.4 | PRESERVATIVE |
| PURIFIED WATER | 45.8 | SOLVENT |
| LECITHIN LIQUID | 0.8 | EMULSIFIER |
| OLIVE OIL | 5.0 | SKIN CONDITIONER |
| JOJOBA OIL | 3.1 | SKIN CONDITIONER |
| EVENING PRIMROSE OIL | 1.9 | SKIN CONDITIONER |
| AVOCADO OIL | 1.9 | SKIN CONDITIONER |
| ALOE VERA 1-1 | 4.0 | HERBAL ACTIVE |
| *CENTIPEDA* PLANT EXTRACT 1-1 | 11.7 | HERBAL ACTIVE |
| CALENDULA EXTRACT 1-1 | 3.0 | HERBAL ACTIVE |
| WITCH HAZEL EXTRACT 1-1 | 1.2 | HERBAL ACTIVE |
| EYEBRIGHT EXTRACT 1-1 | 3.6 | HERBAL ACTIVE |
| LAURYL ALCOHOL | 0.5 | EMULSIFIER |
| FRACTIONATED COCONUT OIL | 0.4 | SUSPENSION AGENT |
| TOTAL | 100.0 | |

### Example 22

### EYE MAKE-UP REMOVER

The *Centipeda* plant extract of Example 1 was mixed with the ingredients listed below. This composition can be applied topically and is suitable for use as an eye make-up remover.

| **INGREDIENTS** | **%** | **FUNCTION** |
|---|---|---|
| LANOLIN ALCOHOLS | 1.8 | EMULSIFIER |
| STEARIC ACID | 3.2 | EMULSIFIER |
| CETYL STEARYL ALCOHOL | 1.2 | SKIN CONDITIONER |
| OLIVE OIL | 4.0 | SKIN CONDITIONER |
| METHYL HYDROXYBENZOATE | 0.2 | PRESERVATIVE |
| GLYCERIN | 5.0 | HUMECTANT |
| PURIFIED WATER | 55.4 | SOLVENT |
| ALOE VERA | 16.0 | HERBAL ACTIVE |
| *CENTIPEDA* PLANT EXTRACT 1-1 | 5.0 | HERBAL ACTIVE |
| LAURYL ALCOHOL | 0.5 | EMULSIFIER |
| FRACTIONATED COCONUT OIL | 0.4 | SUSPENSION AGENT |
| ALMOND OIL | 1.7 | SKIN CONDITIONER |
| APRICOT KERNEL OIL | 2.4 | SKIN CONDITIONER |
| ELDER FLOWER EXTRACT 1-1 | 2.1 | HERBAL ACTIVE |
| HONEY | 1.1 | EMOLLIENT |
| TOTAL | 100.0 | |

### Example 23

### HYDRATING GEL MASK

### For Dry Skin

The *Centipeda* plant extract of Example 1 was mixed with the ingredients listed below. This composition can be applied topically and is suitable for use as a hydrating gel mask for dry skin.

| **INGREDIENTS** | **%** | **FUNCTION** |
|---|---|---|
| PURIFIED WATER | 22.7 | SOLVENT |
| ETHANOL | 12.5 | SOLVENT |
| FRACTIONATED COCONUT OIL | 0.5 | SUSPENSION AGENT |
| LAURYL ALCOHOL | 0.6 | EMULSIFIER |
| GLYCERIN | 10.0 | HUMECTANT |
| ALOE VERA | 20.0 | HERBAL ACTIVE |
| OLIVE OIL | 3.1 | SKIN CONDITIONER |
| HONEY | 1.5 | EMOLLIENT |
| LECITHIN | 1.1 | ANTIOXIDANT |
| KAOLIN | 15.0 | ACTIVE |
| PURIFIED TALC | 4.2 | ABSORBING AGENT |
| CUCUMBER EXTRACT 1-1 | 2.5 | HERBAL ACTIVE |
| *CENTIPEDA* PLANT EXTRACT 1-1 | 6.0 | HERBAL ACTIVE |
| METHYL HYDROXYBENZOATE | 0.2 | PRESERVATIVE |
| FENNEL OIL | 0.1 | ESSENTIAL OIL |
| TOTAL | 100.0 | |

### Example 24

### ASTRINGENT LOTION

### For Dry Skin

The *Centipeda* plant extract of Example 1 was mixed with the ingredients listed below. This composition can be applied topically and is suitable for use as an astringent lotion for dry skin.

| **INGREDIENTS** | **%** | **FUNCTION** |
|---|---|---|
| ETHANOL | 35.0 | SOLVENT |
| PURIFIED WATER | 31.5 | SOLVENT |
| GLYCERIN | 5.0 | HUMECTANT |
| HYDROGENATED CASTOR OIL | 0.9 | SUSPENSION AGENT |
| MENTHOL CRYSTALS | 0.1 | SKIN FRESHENER |
| FRUIT ACIDS OF GRAPE, LEMON | 1.6 | ANTIOXIDANT |
| LEMON OIL | 0.2 | ESSENTIAL OIL |
| LIME OIL | 0.4 | ESSENTIAL OIL |
| GERANIUM OIL | 0.2 | ESSENTIAL OIL |
| METHYL HYDROXYBENZOATE | 0.1 | PRESERVATIVE |
| *CENTIPEDA* PLANT EXTRACT 1-1 | 5.0 | HERBAL ACTIVE |
| ALOE VERA | 20.0 | HERBAL ACTIVE |
| TOTAL | 100.0 | |

### Example 25

### NIGHT CREAM

### For Dry Skin

The *Centipeda* plant extract of Example 1 was mixed with the ingredients listed below. This composition can be applied topically and is suitable for use as a night cream for dry skin.

| **INGREDIENTS** | **%** | **FUNCTION** |
|---|---|---|
| CETYL STEARYL ALCOHOL | 10.0 | SKIN CONDITIONER |
| CETOMACROGOL 1000 | 1.6 | EMULSIFIER |
| METHYL HYDROXYBENZOATE | 0.3 | PRESERVATIVE |
| GRAPESEED OIL | 8.0 | SKIN CONDITIONER |
| GLYCERIN | 7.0 | HUMECTANT |
| PURIFIED WATER | 45.0 | SOLVENT |
| *CENTIPEDA* PLANT EXTRACT 1-1 | 6.0 | HERBAL ACTIVE |
| ROSEMARY EXTRACT 1-1 | 1.0 | HERBAL ACTIVE |
| ALOE VERA | 10.0 | HERBAL ACTIVE |
| ALMOND OIL | 1.7 | SKIN CONDITIONER |
| AVOCADO OIL | 2.1 | SKIN CONDITIONER |
| OLIVE OIL | 6.0 | SKIN CONDITIONER |
| LEMON GRASS OIL | 0.4 | ESSENTIAL OIL |
| LAURYL ALCOHOL | 0.5 | EMULSIFIER |
| FRACTIONATED COCONUT OIL | 0.4 | SUSPENSION AGENT |
| TOTAL | 100.0 | |

### Example 26

### DAY CREAM

### For Dry Skin

The *Centipeda* plant extract of Example 1 was mixed with the ingredients listed below. This composition can be applied topically and is suitable for use as a day cream for dry skin.

| **INGREDIENTS** | **%** | **FUNCTION** |
|---|---|---|
| CETYL STEARYL ALCOHOL | 8.4 | SKIN CONDITIONER |
| CETOMACROGOL 1000 | 1.6 | EMULSIFIER |
| GRAPESEED OIL | 6.5 | SKIN CONDITIONER |
| OLIVE OIL | 2.0 | SKIN CONDITIONER |
| METHYL HYDROXYBENZOATE | 0.4 | PRESERVATIVE |
| GLYCERIN | 8.0 | HUMECTANT |
| PURIFIED WATER | 52.9 | SOLVENT |
| *CENTIPEDA* PLANT EXTRACT 1-1 | 5.0 | HERBAL ACTIVE |
| ALOE VERA | 10.0 | HERBAL ACTIVE |
| ORANGE BLOSSOM | 0.4 | ESSENTIAL OIL |
| LETTUCE EXTRACT 1-1 | 2.0 | HERBAL ACTIVE |
| CALENDULA EXTRACT 1-1 | 1.9 | HERBAL ACTIVE |
| LAURYL ALCOHOL | 0.5 | EMULSIFIER |
| FRACTIONATED COCONUT OIL | 0.4 | SUSPENSION AGENT |
| TOTAL | 100.0 | |

### Example 27

### TONER

### For Dry Skin

The *Centipeda* plant extract of Example 1 was mixed with the ingredients listed below. This composition can be applied topically and is suitable for use as a toner for dry skin.

| **INGREDIENTS** | **%** | **FUNCTION** |
|---|---|---|
| PURIFIED WATER | 42.9 | SOLVENT |
| ETHANOL | 20.0 | SOLVENT |
| HYDROGENATED CASTOR OIL | 1.0 | SUSPENSION AGENT |
| ALOE VERA JUICE | 25.0 | HERBAL ACTIVE |
| *CENTIPEDA* PLANT EXTRACT 1-1 | 5.0 | HERBAL ACTIVE |
| CUCUMBER EXTRACT 1-1 | 2.0 | HERBAL ACTIVE |
| CALENDULA EXTRACT 1-1 | 2.0 | HERBAL ACTIVE |
| CALENDULA OIL | 1.0 | SKIN CONDITIONER |
| AVOCADO OIL | 1.0 | SKIN CONDITIONER |
| FENNEL OIL | 0.1 | ESSENTIAL OIL |
| TOTAL | 100.0 | |

### Example 28

### CLEANSER LOTION

### For Dry Skin

The *Centipeda* plant extract of Example 1 was mixed with the ingredients listed below. This composition can be applied topically and is suitable for use as a cleansing lotion for dry skin.

| **INGREDIENTS** | **%** | **FUNCTION** |
|---|---|---|
| LANOLIN ALCOHOLS | 1.8 | EMULSIFIER |
| STEARIC ACID | 3.2 | EMULSIFIER |
| CETYL STEARYL ALCOHOL | 1.2 | SKIN CONDITIONER |
| GRAPESEED OIL | 5.0 | SKIN CONDITIONER |
| METHYL HYDROXYBENZOATE | 0.2 | PRESERVATIVE |
| GLYCERIN | 2.0 | HUMECTANT |
| PURIFIED WATER | 66.6 | SOLVENT |
| ALOE VERA | 8.0 | HERBAL ACTIVE |
| *CENTIPEDA* PLANT EXTRACT | 5.0 | HERBAL ACTIVE |
| LAURYL ALCOHOL | 0.5 | EMULSIFIER |
| FRACTIONATED COCONUT OIL | 0.4 | SUSPENSION AGENT |
| CUCUMBER EXTRACT 1-1 | 3.0 | HERBAL ACTIVE |
| CALENDULA EXTRACT 1-1 | 2.9 | HERBAL ACTIVE |
| GRAPEFRUIT OIL | 0.2 | ESSENTIAL OIL |
| TOTAL | 100.0 | |

### Example 29

### ASTRINGENT LOTION

### For Normal To Oily Skin

The *Centipeda* plant extract of Example 1 was mixed with the ingredients listed below. This composition can be applied topically and is suitable for use as an astringent lotion for normal to oily skin.

| **INGREDIENTS** | **%** | **FUNCTION** |
|---|---|---|
| ETHANOL | 40.0 | SOLVENT |
| PURIFIED WATER | 31.5 | SOLVENT |
| HYDROGENATED CASTOR OIL | 0.9 | SUSPENSION AGENT |
| METHANOL CRYSTALS | 0.1 | SKIN FRESHENER |
| FRUIT ACIDS OF GRAPE, LEMON | 1.6 | ANTIOXIDANT |
| LEMON OIL | 0.2 | ESSENTIAL OIL |
| GRAPEFRUIT OIL | 0.4 | ESSENTIAL OIL |
| WATTLE OIL | 0.2 | ESSENTIAL OIL |
| METHYL HYDROXYBENZOATE | 0.1 | PRESERVATIVE |
| *CENTIPEDA* PLANT EXTRACT 1-1 | 5.0 | HERBAL ACTIVE |
| ALOE VERA | 20.0 | HERBAL ACTIVE |
| TOTAL | 100.0 | |

### Example 30

### PURIFYING GEL MASK

### For Normal To Oily Skin

The *Centipeda* plant extract of Example 1 was mixed with the ingredients listed below. This composition can be applied topically and is suitable for use as purifying gel mask for normal to oily skin.

| **INGREDIENTS** | **%** | **FUNCTION** |
|---|---|---|
| PURIFIED WATER | 35.0 | SOLVENT |
| ETHANOL | 12.3 | SOLVENT |
| FRACTIONATED COCONUT OIL | 0.5 | SUSPENSION AGENT |
| LAURYL ALCOHOL | 0.6 | EMULSIFIER |
| GLYCERIN | 5.0 | HUMECTANT |
| ALOE VERA | 15.0 | HERBAL ACTIVE |
| HONEY | 2.1 | EMOLLIENT |
| LECITHIN | 1.3 | ANTIOXIDANT |
| KAOLIN | 15.0 | ACTIVE |
| PURIFIED TALC | 4.2 | ABSORBING AGENT |
| WITCH HAZEL EXTRACT 1-1 | 3.0 | HERBAL ACTIVE |
| LEMON OIL | 0.1 | ESSENTIAL OIL |
| *CENTIPEDA* PLANT EXTRACT 1-1 | 5.7 | HERBAL ACTIVE |
| METHYL HYDROXYBENZOATE | 0.2 | PRESERVATIVE |
| TOTAL | 100.0 | |

### Example 31

### NIGHT CREAM

### For Normal To Oily Skin

The *Centipeda* plant extract of Example 1 was mixed with the ingredients listed below. This composition can be applied topically and is suitable for use as a night cream for normal to oily skin.

| **INGREDIENTS** | **%** | **FUNCTION** |
|---|---|---|
| CETYL STEARYL ALCOHOL | 10.0 | SKIN CONDITIONER |
| CETOMACROGOL 1000 | 1.6 | EMULSIFIER |
| METHYL HYDROXYBENZOATE | 0.4 | PRESERVATIVE |
| GRAPESEED OIL | 11.6 | SKIN CONDITIONER |
| GLYCERIN | 5.0 | HUMECTANT |
| PURIFIED WATER | 50.0 | SOLVENT |
| *CENTIPEDA* PLANT EXTRACT 1-1 | 6.0 | HERBAL ACTIVE |
| GENTIAN EXTRACT 1-1 | 1.4 | HERBAL ACTIVE |
| ALOE VERA | 7.0 | HERBAL ACTIVE |
| ALMOND OIL | 1.2 | SKIN CONDITIONER |
| AVOCADO OIL | 1.5 | SKIN CONDITIONER |
| OLIVE OIL | 3.0 | SKIN CONDITIONER |
| GRAPEFRUIT OIL | 0.4 | ESSENTIAL OIL |
| LAURYL ALCOHOL | 0.5 | EMULSIFIER |
| FRACTIONATED COCONUT OIL | 0.4 | SUSPENSION AGENT |
| TOTAL | 100.0 | |

### Example 32

### DAY CREAM

### For Normal To Oily Skin

The *Centipeda* plant extract of Example 1 was mixed with the ingredients listed below. This composition can be applied topically and is suitable for use as a day cream for normal to oily skin.

| **INGREDIENTS** | **%** | **FUNCTION** |
|---|---|---|
| CETYL STEARYL ALCOHOL | 8.4 | SKIN CONDITIONER |
| CETOMACROGOL 1000 | 1.6 | EMULSIFIER |
| GRAPESEED OIL | 8.0 | SKIN CONDITIONER |
| METHYL HYDROXYBENZOATE | 0.4 | PRESERVATIVE |
| GLYCERIN | 3.0 | HUMECTANT |
| PURIFIED WATER | 58.6 | SOLVENT |
| *CENTIPEDA* PLANT EXTRACT 1-1 | 5.0 | HERBAL ACTIVE |
| ALOE VERA | 10.0 | HERBAL ACTIVE |
| WATTLE OIL | 0.3 | ESSENTIAL OIL |
| CALENDULA EXTRACT 1-1 | 2.8 | HERBAL ACTIVE |
| SESAME OIL | 1.0 | SKIN CONDITIONER |
| LAURYL ALCOHOL | 0.5 | EMULSIFIER |
| FRACTIONATED COCONUT OIL | 0.4 | SUSPENSION AGENT |
| TOTAL | 100.0 | |

### Example 33

### TONER

### For Normal To Oily Skin

The *Centipeda* plant extract of Example 1 was mixed with the ingredients listed below. This composition can be applied topically and is suitable for use as a toner for normal to oily skin.

| **INGREDIENTS** | **%** | **FUNCTION** |
|---|---|---|
| PURIFIED WATER | 46.0 | SOLVENT |
| ETHANOL | 20.0 | SOLVENT |
| HYDROGENATED CASTOR OIL | 1.0 | SUSPENSION AGENT |
| WITCH HAZEL EXTRACT 1-1 | 5.0 | HERBAL ACTIVE |
| ALOE VERA JUICE | 20.0 | HERBAL ACTIVE |
| *CENTIPEDA* PLANT EXTRACT 1-1 | 5.0 | HERBAL ACTIVE |
| LIME OIL | 0.2 | ESSENTIAL OIL |
| RED CLOVER EXTRACT 1-1 | 2.8 | HERBAL ACTIVE |
| TOTAL | 100.0 | |

### Example 34

### CLEANSER LOTION

### For Normal To Oily Skin

The *Centipeda* plant extract of Example 1 was mixed with the ingredients listed below. This composition can be applied topically and is suitable for use as a cleansing lotion for normal to oily skin.

| **INGREDIENTS** | **%** | **FUNCTION** |
|---|---|---|
| LANOLIN ALCOHOLS | 1.8 | EMULSIFIER |
| STEARIC ACID | 3.2 | EMULSIFIER |
| CETYL STEARYL ALCOHOL | 1.2 | SKIN CONDITIONER |
| GRAPESEED OIL | 5.0 | SKIN CONDITIONER |
| METHYL HYDROXYBENZOATE | 0.2 | PRESERVATIVE |
| GLYCERIN | 2.0 | HUMECTANT |
| PURIFIED WATER | 55.5 | SOLVENT |
| ALOE VERA | 8.0 | HERBAL ACTIVE |
| *CENTIPEDA* PLANT EXTRACT 1-1 | 5.0 | HERBAL ACTIVE |
| LAURYL ALCOHOL | 0.5 | EMULSIFIER |
| FRACTIONATED COCONUT OIL | 0.4 | SUSPENSION AGENT |
| CHAMOMILE EXTRACT 1-1 | 3.2 | HERBAL ACTIVE |
| CALENDULA EXTRACT 1-1 | 2.7 | HERBAL ACTIVE |
| WATTLE OIL | 0.2 | ESSENTIAL OIL |
| TOTAL | 100.0 | |

### Example 35

### FOAMING CLEANSER LOTION

The *Centipeda* plant extract of Example 1 was mixed with the ingredients listed below. This composition can be applied topically and is suitable for use as a foaming cleanser lotion.

| **INGREDIENTS** | **%** | **FUNCTION** |
|---|---|---|
| FRACTIONATED COCONUT OIL | 10.0 | SUSPENSION AGENT |
| COCO-BETAINE | 9.0 | ANTISTATIC AGENT |
| HONEY | 1.0 | EMOLLIENT |
| GLYCERIN | 10.0 | HUMECTANT |
| SODIUM LAURYL ETHER SULPHATE | 7.0 | WETTING AGENT |
| METHYL HYDROXYBENZOATE | 0.2 | PRESERVATIVE |
| ALOE VERA | 8.0 | HERBAL ACTIVE |
| LEMON OIL | 0.2 | ESSENTIAL OIL |
| TEA TREE OIL | 0.1 | ESSENTIAL OIL |
| PURIFIED WATER | 46.5 | SOLVENT |
| *CENTIPEDA* PLANT EXTRACT 1-1 | 4.0 | HERBAL ACTIVE |
| TOTAL | 100.0 | |

### Example 36

### HAIR AND SCALP MOISTURISING AND CLEANSING TREATMENT

The *Centipeda* plant extract of Example 1 was mixed with the ingredients listed below. This composition can be applied topically and is suitable for use as a hair and scalp moisturising and cleansing treatment.

| **INGREDIENTS** | **%** | **FUNCTION** |
|---|---|---|
| JOJOBA OIL | 2.0 | HAIR CONDITIONER |
| PURIFIED WATER | 46.0 | SOLVENT |
| GLYCERIN | 5.0 | HUMECTANT |
| SODIUM LAURYL ETHER SULPHATE | 5.0 | EMULSIFYING AGENT |
| FRACTIONATED COCONUT OIL | 6.0 | SUSPENSION AGENT |
| COCOAMIDOPROPYL BETAINE | 4.0 | WETTING AGENT |
| CETYL STEARYL ALCOHOL | 2.5 | SKIN CONDITIONER |
| VIRGIN OLIVE OIL | 3.0 | SKIN/HAIR CONDITIONER |
| LANOLIN WAX | 1.5 | EMULSIFIER |
| HONEY | 1.0 | EMOLLIENT |
| ALOE VERA | 8.0 | HERBAL ACTIVE |
| LECITHIN LIQUID | 0.5 | ANTIOXIDANT |
| ROSEMARY EXTRACT 1-1 | 3.0 | HERBAL ACTIVE |
| SAGE EXTRACT 1-1 | 2.0 | HERBAL ACTIVE |
| CHAMOMILE EXTRACT 1-1 | 1.7 | HERBAL ACTIVE |
| HORSE CHESTNUT EXTRACT 1-1 | 2.0 | HERBAL ACTIVE |
| LAVENDER OIL | 0.5 | ESSENTIAL OIL |
| WATTLE OIL | 0.3 | ESSENTIAL OIL |
| METHYL HYDROXYBENZOATE | 1.0 | PRESERVATIVE |
| *CENTIPEDA* PLANT EXTRACT 1-1 | 5.0 | HERBAL ACTIVE |
| TOTAL | 100.0 | |

### Example 37

### TREATMENT CONDITIONER

### Henna & Rosemary

The *Centipeda* plant extract of Example 1 was mixed with the ingredients listed below. This composition can be applied topically and is suitable for use as a treatment conditioner.

| **INGREDIENTS** | **%** | **FUNCTION** |
|---|---|---|
| CETYL STEARYL ALCOHOL | 6.0 | SKIN CONDITIONER |
| STEARYL ALCOHOL | 3.0 | EMULSIFIER |
| METHYL HYDROXYBENZOATE | 0.2 | PRESERVATIVE |
| OLIVE OIL | 4.69 | SKIN CONDITIONER |
| ALOE VERA | 6.0 | HERBAL ACTIVE |
| ROSEMARY EXTRACT | 3.0 | HERBAL ACTIVE |
| *CENTIPEDA* PLANT EXTRACT 1-1 | 7.0 | HERBAL ACTIVE |
| HENNA EXTRACT | 4.0 | HERBAL ACTIVE |
| PURIFIED WATER | 66.0 | SOLVENT |
| ROSEMARY OIL | 0.05 | ESSENTIAL OIL |
| LAVENDER OIL | 0.05 | ESSENTIAL OIL |
| TEA TREE OIL | 0.01 | ESSENTIAL OIL |
| TOTAL | 100.0 | |

### Example 38

### CONDITIONER

The *Centipeda* plant extract of Example 1 was mixed with the ingredients listed below. This composition can be applied topically and is suitable for use as a conditioner.

| **INGREDIENTS** | **%** | **FUNCTION** |
|---|---|---|
| CETYL STEARYL ALCOHOL | 6.0 | SKIN CONDITIONER |
| STEARYL ALCOHOL | 3.0 | EMULSIFIER |
| METHYL HYDROXYBENZOATE | 0.2 | PRESERVATIVE |
| OLIVE OIL | 4.69 | SKIN CONDITIONER |
| ALOE VERA | 10.0 | HERBAL ACTIVE |
| *CENTIPEDA* PLANT EXTRACT 1-1 | 7.0 | HERBAL ACTIVE |
| PURIFIED WATER | 69.0 | SOLVENT |
| SANDALWOOD OIL | 0.02 | ESSENTIAL OIL |
| GERANIUM OIL | 0.02 | ESSENTIAL OIL |
| YLANG YLANG OIL | 0.01 | ESSENTIAL OIL |
| LAVENDER OIL | 0.05 | ESSENTIAL OIL |
| TEA TREE OIL | 0.01 | ESSENTIAL OIL |
| TOTAL | 100.0 | |

### Example 39

### SHAMPOO

### For Dry Hair

The *Centipeda* plant extract of Example 1 was mixed with the ingredients listed below. This composition can be applied topically and is suitable for use as a shampoo for dry hair.

| **INGREDIENTS** | **%** | **FUNCTION** |
|---|---|---|
| SODIUM LAURYL ETHER SULPHATE | 12.0 | EMULSIFYING AGENT |
| FRACTIONATED COCONUT OIL | 8.0 | SUSPENSION AGENT |
| COCOAMIDOPROPYL BETAINE | 11.0 | WETTING AGENT |
| GLYCERIN | 10.0 | HUMECTANT |
| OLIVE OIL | 3.0 | CONDITIONING AGENT |
| PURIFIED WATER | 40.615 | SOLVENT |
| METHYL HYDROXYBENZOATE | 0.2 | PRESERVATIVE |
| ALOE VERA | 8.0 | HERBAL ACTIVE |
| *CENTIPEDA* PLANT EXTRACT 1-1 | 7.0 | HERBAL ACTIVE |
| SANDALWOOD OIL | 0.005 | ESSENTIAL OIL |
| GERANIUM OIL | 0.02 | ESSENTIAL OIL |
| YLANG YLANG OIL | 0.01 | ESSENTIAL OIL |
| LAVENDER OIL | 0.05 | ESSENTIAL OIL |
| TEA TREE OIL | 0.01 | ESSENTIAL OIL |
| TOTAL | 100.0 | |

### Example 40

### SHAMPOO

### For Normal Hair

The *Centipeda* plant extract of Example 1 was mixed with the ingredients listed below. This composition can be applied topically and is suitable for use as a shampoo for normal hair.

| **INGREDIENTS** | **%** | **FUNCTION** |
|---|---|---|
| SODIUM LAURYL ETHER SULPHATE | 15.0 | EMULSIFYING AGENT |
| FRACTIONATED COCONUT OIL | 8.0 | SUSPENSION AGENT |
| COCOAMIDOPROPYL BETAINE | 11.0 | WETTING AGENT |
| GLYCERIN | 8.0 | HUMECTANT |
| PURIFIED WATER | 40.615 | SOLVENT |
| METHYL HYDROXYBENZOATE | 0.2 | PRESERVATIVE |
| ALOE VERA | 10.0 | HERBAL ACTIVE |
| *CENTIPEDA* PLANT EXTRACT 1-1 | 7.0 | HERBAL ACTIVE |
| SANDALWOOD OIL | 0.005 | ESSENTIAL OIL |
| GERANIUM OIL | 0.02 | ESSENTIAL OIL |
| YLANG YLANG OIL | 0.01 | ESSENTIAL OIL |
| LAVENDER OIL | 0.05 | ESSENTIAL OIL |
| TEA TREE OIL | 0.01 | ESSENTIAL OIL |
| TOTAL | 100.0 | |

### Example 41

### HAND CREAM

### Hand Protectant Cream

The *Centipeda* plant extract of Example 1 was mixed with the ingredients listed below. This composition can be applied topically and is suitable for use as a hand cream.

| **INGREDIENTS** | **%** | **FUNCTION** |
|---|---|---|
| CETYL STEARYL ALCOHOL | 10.0 | SKIN CONDITIONER |
| CETOMACROGOL 1000 | 2.0 | EMULSIFIER |
| OLIVE OIL | 5.0 | SKIN CONDITIONER |
| DIMETHICONE | 3.0 | SKIN PROTECTANT |
| LANOLIN WAX | 2.5 | EMULSIFIER |
| COCOA BUTTER | 1.0 | SKIN CONDITIONER |
| BEESWAX | 1.0 | EMULSIFIER |
| GLYCERIN | 5.1 | HUMECTANT |
| METHYL HYDROXYBENZOATE | 0.3 | PRESERVATIVE |
| PURIFIED WATER | 50.0 | SOLVENT |
| *CENTIPEDA* PLANT EXTRACT 3-1 | 5.0 | HERBAL ACTIVE |
| ALOE VERA | 5.6 | HERBAL ACTIVE |
| HORSETAIL EXTRACT 2-1 | 4.1 | HERBAL ACTIVE |
| MARSHMALLOW EXTRACT 301 | 3.5 | HERBAL ACTIVE |
| LEMON OIL | 1.0 | ESSENTIAL OIL |
| LAURYL ALCOHOL | 0.5 | EMULSIFIER |
| FRACTIONATED COCONUT OIL | 0.4 | SUSPENSION AGENT |
| TOTAL | 100.0 | |

### Example 42

### HAND AND NAIL CREAM

The *Centipeda* plant extract of Example 1 was mixed with the ingredients listed below. This composition can be applied topically and is suitable for use as a hand and nail cream.

| **INGREDIENTS** | **%** | **FUNCTION** |
|---|---|---|
| CETYL STEARYL ALCOHOL | 10.0 | SKIN CONDITIONER |
| CETOMACROGOL 1000 | 1.6 | EMULSIFIER |
| OLIVE OIL | 10.0 | SKIN CONDITIONER |
| LANOLIN WAX | 2.0 | EMULSIFIER |
| GLYCERIN | 5.0 | HUMECTANT |
| METHYL HYDROXYBENZOATE | 0.4 | PRESERVATIVE |
| PURIFIED WATER | 46.4 | SOLVENT |
| ALOE VERA | 7.0 | HERBAL ACTIVE |
| *CENTIPEDA* PLANT EXTRACT 3-1 | 3.0 | HERBAL ACTIVE |
| JOJOBA OIL | 2.0 | SKIN CONDITIONER |
| DIMETHICONE | 1.0 | SKIN PROTECTANT |
| HORSETAIL EXTRACT 1-1 | 1.4 | HERBAL ACTIVE |
| ST. JOHNS WORT EXTRACT 1.1 | 2.3 | HERBAL ACTIVE |
| CALENDULA EXTRACT 1-1 | 4.6 | HERBAL ACTIVE |
| CALENDULA OIL | 0.9 | SKIN CONDITIONER |
| GRAPEFRUIT OIL | 0.5 | ESSENTIAL OIL |
| MARSHMALLOW EXTRACT 1-1 | 1.0 | BIOLOGICAL ADDITION |
| LAURYL ALCOHOL | 0.5 | EMULSIFIER |
| FRACTIONATED COCONUT OIL | 0.4 | SUSPENSION AGENT |
| TOTAL | 100.0 | |

### Example 43

### BODY LOTION

The *Centipeda* plant extract of Example 1 was mixed with the ingredients listed below. This composition can be applied topically and is suitable for use as a body lotion.

| **INGREDIENTS** | **%** | **FUNCTION** |
|---|---|---|
| EVENING PRIMROSE OIL | 4.6 | SKIN CONDITIONER |
| OLIVE OIL | 2.25 | SKIN CONDITIONER |
| LANOLIN ALCOHOLS | 1.75 | EMULSIFIER |
| STEARIC ACID | 3.3 | EMULSIFIER |
| EVENING PRIMROSE OIL | 4.6 | SKIN CONDITIONER |
| OLIVE OIL | 2.25 | SKIN CONDITIONER |
| LANOLIN ALCOHOLS | 1.75 | EMULSIFIER |
| STEARIC ACID | 3.3 | EMULSIFIER |
| CETYL STEARYL ALCOHOL | 1.1 | SKIN CONDITIONER |
| METHYL HYDROXYBENZOATE | 0.3 | PRESERVATIVE |
| GLYCERIN | 5.0 | HUMECTANT |
| PURIFIED WATER | 61.7 | SOLVENT |
| ALOE VERA 2-1 | 13.4 | HERBAL ACTIVE |
| *CENTIPEDA* PLANT EXTRACT 2-1 | 5.0 | HERBAL ACTIVE |
| TEA TREE OIL | 0.5 | ESSENTIAL OIL |
| SANDALWOOD OIL | 0.01 | ESSENTIAL OIL |
| GERANIUM OIL | 0.07 | ESSENTIAL OIL |
| YLANG YLANG OIL | 0.02 | ESSENTIAL OIL |
| LAVENDER OIL | 0.1 | ESSENTIAL OIL |
| LAURYL ALCOHOL | 0.5 | EMULSIFIER |
| FRACTIONATED COCONUT OIL | 0.4 | SUSPENSION AGENT |
| TOTAL | 100.0 | |

### Example 44

### BODY SCRUB

The *Centipeda* plant extract of Example 1 was mixed with the ingredients listed below. This composition can be applied topically and is suitable for use as a body scrub.

| **INGREDIENTS** | **%** | **FUNCTION** |
|---|---|---|
| LANOLIN ALCOHOL | 1.3 | EMULSIFIER |
| OLIVE OIL | 3.2 | SKIN CONDITIONER |
| GRAPESEED OIL | 5.6 | SKIN CONDITIONER |
| STEARIC ACID | 3.7 | EMULSIFIER |
| CETYL STEARYL ALCOHOL | 1.9 | SKIN CONDITIONER |
| METHYL HYDROXYBENZOATE | 0.3 | PRESERVATIVE |
| FRACTIONATED COCONUT OIL | 8.3 | SUSPENSION AGENT |
| COCAMIDOPROPYL BETAINE | 4.7 | WETTING AGENT |
| LECITHIN | 1.2 | ANTIOXIDANT |
| ALOE VERA | 8.1 | HERBAL ACTIVE |
| LEMON OIL | 0.7 | ESSENTIAL OIL |
| AVOCADO OIL | 1.4 | SKIN CONDITIONER |
| WHEATGERM OIL | 0.8 | SKIN CONDITIONER |
| GLYCERIN | 4.3 | HUMECTANT |
| *CENTIPEDA* PLANT EXTRACT 1-1 | 8.7 | HERBAL ACTIVE |
| SARSAPARILLA HERB | 0.8 | HERBAL ACTIVE |
| EVENING PRIMROSE SEEDS | 0.6 | HERBAL ACTIVE |
| PURIFIED WATER | 44.4 | SOLVENT |
| TOTAL | 100.0 | |

### Example 45

### VITAMIN "E" CREAM

The *Centipeda* plant extract of Example 1 was mixed with the ingredients listed below. This composition can be_applied topically and is suitable for use as a vitamin "E" cream.

| **INGREDIENTS** | **%** | **FUNCTION** |
|---|---|---|
| PURIFIED WATER | 50.0 | SOLVENT |
| PARAFFIN SOFT WHITE | 3.1 | SKIN CONDITIONER |
| CETOMACROGOL 1000 | 1.2 | EMULSIFIER |
| LANOLIN WAX | 1.0 | EMULSIFIER |
| GRAPESEED OIL | 8.7 | SKIN CONDITIONER |
| GLYCERIN | 10.2 | HUMECTANT |
| METHYL HYDROXYBENZOATE | 0.2 | PRESERVATIVE |
| LAURYL ALCOHOL | 0.5 | EMULSIFIER |
| FRACTIONATED COCONUT OIL | 0.4 | SUSPENSION AGENT |
| ALOE VERA | 10.0 | HERBAL ACTIVE |
| APRICOT KERNEL OIL | 2.7 | SKIN CONDITIONER |
| TOCOPHEROL (VITAMIN E) | 3.9 | ANTIOXIDANT |
| *CENTIPEDA* PLANT EXTRACT 1-1 | 8.1 | HERBAL ACTIVE |
| TOTAL | 100.0 | |

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

## Claims

1. A composition for the treatment and prophylaxis of medical conditions of a human or other animal, comprising an aqueous alcoholic extract from the plant *Centipeda Cunninghamii,* together with an acceptable carrier and/or diluent, wherein the aqueous alcoholic extract is an extract of plant material with aqueous ethanol in the range of from 10% ethanol/90% water to 95% ethanol/5% water.

2. A composition according to claim 1 wherein the aqueous alcoholic extract is an extract in 30% ethanol/70% water.

3. A composition according to claim 1 or 2 wherein the extract is prepared from part or all of the plant.

4. A composition according to any one of claims 1 to 3, wherein the extract is prepared by maceration, percolation, infusion, decoction, oil infusion or alcohol/ethanol extraction.

5. A composition according to any one of claims 1 to 4, wherein the extract is prepared from dried plant material.

6. A composition according to claim 1, wherein the carrier and/or diluent is an orally or topically acceptable carrier and/or diluent.

7. A composition according to claim 6, wherein the carrier and/or diluent comprises one or more of:
emulsifiers, skin protectants, skin conditioners, humectants, preservatives, solvents, herbal actives, essential oils, suspension agents, anionic emulsifying agents, astringents, antiseptics, healing agents, germicides, anti-inflammatories, skin fresheners, wetting agents, antioxidants, skin lubricants, emollients, absorbing agents, preservatives, skin/hair conditioners, abrasives, detergents, binders and/or deodorizing agents, solvents, dispersion media, fillers, solid carriers, aqueous solutions, coatings, antibacterial and antifungal agents, isotonic or absorption delaying agents.

8. A composition according to any one of the preceding claims, in the form of an ingestible tablet, buccal tablet, capsule, lozenge, cachet, wafer, a suspension in an aqueous liquid, a syrup, an elixir, an emulsion, a mouth lotion, mouth gel or gargle solution, a liquid or semi-liquid preparation, a cream, a homogeneous viscous or semi-solid emulsion, a gel, a liniment, a lotion, an ointment, a paint, a paste, a solution, a spray; a stick or a suspension, a deodorant, facial scrub, shaving cream or gel, eye/face cream or gel, make-up remover, toner, cleanser, shampoo, conditioner or breath freshener.

9. Use in the manufacture of a medicament for acne, pimples, herpes, bed sores, skin irritations, cuts, psoriasis, skin sun damage, eczema, nasal irritations, rashes, allergic reactions, inflammations, bacterial infections, gastrointestinal disorders, dermatitis, diuretic treatment, skin proliferation or skin disease treatment of a composition according to claims 1 to 8.

10. A composition for cosmetic treatment of a human or other animal, comprising an aqueous alcoholic extract from the plant *Centipeda Cunninghamii,* together with an acceptable carrier and/or diluent, wherein the aqueous alcoholic extract is an extract of plant material with aqueous ethanol in the range of from 10% ethanol/90% water to 95% ethanol/5% water.

11. A composition according to claim 10 wherein the aqueous alcoholic extract is an extract in 30% ethanol/70% water.

12. A composition according to claim 10 or 11 wherein the extract is prepared from part or all of the plant.

13. A composition according to any one of claims 10 to 12, wherein the extract is prepared by maceration, percolation, infusion, decoction, oil infusion or alcohol/ethanol extraction.

14. A composition according to any one of claims 10 to 13, wherein the extract is prepared from dried plant material.

15. A composition according to claim 10, wherein the carrier and/or diluent is an orally or topically acceptable carrier and/or diluent.

16. A composition according to claim 10, wherein the carrier and/or diluent comprises one or more of:
emulsifiers, skin protectants, skin conditioners, humectants, preservatives, solvents, herbal actives, essential oils, suspension agents, anionic emulsifying agents, astringents, antiseptics, healing agents, germicides, anti-inflammatories, skin fresheners, wetting agents, antioxidants, skin lubricants, emollients, absorbing agents, preservatives, skin/hair conditioners, abrasives, detergents, binders and/or deodorizing agents, solvents, dispersion media, fillers, solid carriers, aqueous solutions, coatings, antibacterial and antifungal agents, isotonic or absorption delaying agents.

17. A composition according to any one of claims 10 to 16, in the form of an ingestible tablet, buccal tablet, capsule, lozenge, cachet, wafer, a suspension in an aqueous liquid, a syrup, an elixir, an emulsion, a mouth lotion, mouth gel or gargle solution, a liquid or semi-liquid preparation, a cream, a homogeneous viscous or semi-solid emulsion, a gel, a liniment, a lotion, an ointment, a paint, a paste, a solution, a spray, a stick or a suspension, a deodorant, facial scrub, shaving cream or gel, eye/face cream or gel, make-up remover, toner, cleanser, shampoo, conditioner or breath freshener.

## Patentansprüche

1. Zusammensetzung zur Behandlung und Vorbeugung von medizinischen Zuständen eines Menschen oder eines anderen Tieres, umfassend einen wässrigen alkoholischen Extrakt aus der Pflanze *Centipeda Cunninghamii,* zusammen mit einem annehmbaren Träger und/oder Verdünnungsmittel, wobei der wässrige alkoholische Extrakt ein Extrakt aus Pflanzenmaterial mit wässrigem Ethanol im Bereich von 10 % Ethanol/90 % Wasser bis 95 % Ethanol/5 % Wasser ist.

2. Zusammensetzung nach Anspruch 1, wobei der wässrige alkoholische Extrakt ein Extrakt in 30 % Ethanol/70 % Wasser ist.

3. Zusammensetzung nach Anspruch 1 oder 2, wobei der Extrakt aus einem Teil der Pflanze oder der gesamten Pflanze hergestellt wird.

4. Zusammensetzung nach einem der Ansprüche 1 bis 3, wobei der Extrakt mittels Mazeration, Perkolation, Infusion, Abkochung, ÖI-Infusion oder alkoholischer/ethanolischer Extraktion hergestellt wird.

5. Zusammensetzung nach einem der Ansprüche 1 bis 4, wobei der Extrakt aus getrocknetem Pflanzenmaterial hergestellt wird.

6. Zusammensetzung nach Anspruch 1, wobei der Träger und/oder das Verdünnungsmittel ein oral oder topisch annehmbarer Träger und/oder ein oral oder topisch annehmbares Verdünnungsmittel darstellt.

7. Zusammensetzung nach Anspruch 6, wobei der Träger und/oder das Verdünnungsmittel eines oder mehrere von:
Emulgatoren, Hautschutzmitteln, Hautconditionern, Feuchthaltemitteln, Konservierungsmitteln, Lösungsmitteln, pflanzlichen Wirkstoffen, ätherischen Ölen, Suspendiermitteln, anionischen Emulgiermitteln, Adstringenzien, Antiseptika, Heilmitteln, keimabtötenden Mitteln, anti-entzündlichen Mitteln, Hauterfrischungsmitteln, Benetzungsmitteln, Antioxidationsmitteln, Hautgleitmitteln, Weichmachern, Absorptionsmitteln, Konservierungsmitteln, Haut-/Haar-Conditionern, Abrasiva, Detergentien, Bindemitteln und/oder geruchsbekämpfenden Mitteln, Lösungsmitteln, Dispersionsmitteln, Füllmitteln, festen Trägern, wässrigen Lösungen, Beschichtungen, antibakteriellen und antimykotischen Mitteln, isotonischen oder Absorptions-verzögernden Mitteln umfasst.

8. Zusammensetzung nach einem der vorhergehenden Ansprüche in Form einer einnehmbaren Tablette, Bukkaltablette, Kapsel, Lutschtablette, Oblate, Waffel, einer Suspension in einer wässrigen Flüssigkeit, einem Sirup, einem Elixier, einer Emulsion, einer Mundlotion, Mundgel oder Gurgellösung, einer flüssigen oder halbflüssigen Zubereitung, einer Creme, einer homogen viskosen oder halb-festen Emulsion, einem Gel, einem Einreibemittel, einer Lotion, einer Salbe, einer Farbe, einer Paste, einer Lösung, einem Spray, einem Stift oder einer Suspension, einem Deodorant, einem Gesichtspeeling, Rasiercreme oder Rasiergel, Augen-/Gesichtscreme oder Augen-/Gesichtsgel, Make-Up-Entferner, Toner, Reinigungsmittel, Shampoo, Conditioner oder Atemerfrischungsmittel.

9. Verwendung einer Zusammensetzung nach Ansprüchen 1 bis 8 zur Herstellung eines Arzneimittels für Akne, Pickel, Herpes, wundgelegene Stellen, Hautreizungen, Schnittwunden, Psoriasis, Hautschäden durch Sonne, Ekzeme, nasale Reizungen, Hautausschläge, allergische Reaktionen, Entzündungen, bakterielle Infektionen, gastrointestinale Erkrankungen, Dermatitis, diuretische Behandlung, Hautproliferation oder Behandlung von Hauterkrankungen.

10. Zusammensetzung zur kosmetischen Behandlung eines Menschen oder eines anderen Tieres, umfassend einen wässrigen alkoholischen Extrakt aus der Pflanze *Centipeda Cunninghamii,* zusammen mit einem annehmbaren Träger und/oder Verdünnungsmittel, wobei der wässrige alkoholische Extrakt ein Extrakt aus Pflanzenmaterial mit wässrigem Ethanol im Bereich von 10 % Ethanol/90 % Wasser bis 95 % Ethanol/5 % Wasser darstellt.

11. Zusammensetzung nach Anspruch 10, wobei der wässrige alkoholische Extrakt ein Extrakt in 30 % Ethanol/70 % Wasser ist.

12. Zusammensetzung nach Anspruch 10 oder 11, wobei der Extrakt aus einem Teil der Pflanze oder der gesamten Pflanze hergestellt wird.

13. Zusammensetzung nach einem der Ansprüche 10 bis 12, wobei der Extrakt mittels Mazeration, Perkolation, Infusion, Abkochung, Öl-Infusion oder alkoholischer/ethanolischer Extraktion hergestellt wird.

14. Zusammensetzung nach einem der Ansprüche 10 bis 13, wobei der Extrakt aus getrocknetem Pflanzenmaterial hergestellt wird.

15. Zusammensetzung nach Anspruch 10, wobei der Träger und/oder das Verdünnungsmittel ein oral oder topisch annehmbarer Träger und/oder ein oral oder topisch annehmbares Verdünnungsmittel ist.

16. Zusammensetzung nach Anspruch 10, wobei der Träger und/oder das Verdünnungsmittel eines oder mehrere von:
Emulgatoren, Hautconditionern, Hautzusatzstoffen, Feuchthaltemitteln, Konservierungsmitteln, Lösungsmitteln, pflanzlichen Wirkstoffen, ätherischen Ölen, Suspendiermitteln, anionischen Emulgiermitteln, Adstringenzien, Antiseptika, Heilmitteln, keimabtötenden Mitteln, anti-entzündlichen Mitteln, Hauterfrischungsmitteln, Benetzungsmitteln, Antioxidationsmitteln, Hautgleitmitteln, Weichmachern, Absorptionsmitteln, Konservierungsmitteln, Haut-/Haar-Conditionern, Abrasiva, Detergentien, Bindemitteln und/oder geruchsbekämpfenden Mitteln, Lösungsmitteln, Dispersionsmitteln, Füllmitteln, festen Trägern, wässrigen Lösungen, Beschichtungen, antibakteriellen und antimykotischen Mitteln, isotonischen oder Absorptions-verzögernden Mitteln umfasst.

17. Zusammensetzung nach einem der Ansprüche 10 bis 16 in Form einer einnehmbaren Tablette, Bukkaltablette, Kapsel, Lutschtablette, Oblate, Waffel, einer Suspension in einer wässrigen Flüssigkeit, einem Sirup, einem Elixier, einer Emulsion, einer Mundlotion, Mundgel oder Gurgellösung, einer flüssigen oder halbflüssigen Zubereitung, einer Creme, einer homogen viskosen oder halb-festen Emulsion, einem Gel, einem Einreibemittel, einer Lotion, einer Salbe, einer Farbe, einer Paste, einer Lösung, einem Spray, einem Stift oder einer Suspension, einem Deodorant, Gesichtspeeling, Rasiercreme oder Rasiergel, Augen-/Gesichtscreme oder Augen-/Gesichtsgel, Make-Up-Entferner, Toner, Reinigungsmittel, Shampoo, Conditioner oder Atemerfrischungsmittel.

## Revendications

1. Composition pour le traitement et la prophylaxie d'affections médicales d'un humain ou d'un autre animal, comprenant un extrait hydro-alcoolique de la plante *Centipeda cunninghamii,* conjointement avec un véhicule et/ou diluant acceptable, dans laquelle l'extrait hydro-alcoolique est un extrait de matière de plante avec de l'éthanol aqueux dans la gamme de 10 % éthanol/90 % eau à 95 % éthanol/5 % eau.

2. Composition selon la revendication 1, dans laquelle l'extrait hydro-alcoolique est un extrait dans 30 % éthanol/70 % eau.

3. Composition selon la revendication 1 ou 2, dans laquelle l'extrait est préparé à partir d'une partie ou de la totalité de la plante.

4. Composition selon l'une quelconque des revendications 1 à 3, dans laquelle l'extrait est préparé par macération, percolation, infusion, décoction, infusion d'huile ou extraction par alcool/éthanol.

5. Composition selon l'une quelconque des revendications 1 à 4, dans laquelle l'extrait est préparé à partir de matière de plante séchée.

6. Composition selon la revendication 1, dans laquelle le véhicule et/ou diluant est un véhicule et/ou diluant oralement ou localement acceptable.

7. Composition selon la revendication 6, dans laquelle le véhicule et/ou diluant comprend un ou plusieurs parmi :
les émulsifiants, les agents protégeant la peau, les agents conditionnant la peau, les humectants, les conservateurs, les solvants, les substances actives d'herbes, les huiles essentielles, les agents de suspension, les agents émulsifiants anioniques, les astringents, les antiseptiques, les agents cicatrisants, les germicides, les anti-inflammatoires, les lotions rafraîchissantes pour la peau, les agents mouillants, les antioxydants, les lubrifiants pour la peau, les émollients, les agents absorbants, les conservateurs, les agents conditionnants pour peau/cheveux, les abrasifs, les détergents, les liants et/ou les agents désodorisants, les solvants, les milieux de dispersion, les charges, les véhicules solides, les solutions aqueuses, les revêtements, les agents antibactériens et antifongiques, les agents isotoniques ou retardant l'absorption.

8. Composition selon l'une quelconque des revendications précédentes, sous la forme d'un comprimé ingérable, d'un comprimé oral, d'une capsule, d'une pastille, d'un cachet, d'une plaquette, d'une suspension dans un liquide aqueux, d'un sirop, d'un élixir, d'une émulsion, d'une lotion buccale, d'un gel buccal ou d'un bain de bouche, d'une préparation liquide ou semi-liquide, d'une crème, d'une émulsion homogène visqueuse ou semi-solide, d'un gel, d'un liniment, d'une lotion, d'une pommade, d'une peinture, d'une pâte, d'une solution, d'une pulvérisation, d'un bâton ou d'une suspension, d'un déodorant, d'un nettoyant pour le visage, d'une crème ou d'un gel à raser, d'une crème ou d'un gel pour les yeux/le visage, d'un démaquillant, d'un tonifiant, d'un nettoyant, d'un shampoing, d'un conditionneur ou d'un rafraîchisseur d'haleine.

9. Utilisation, dans la fabrication d'un médicament d'une composition selon les revendications 1 à 8 pour le traitement de l'acné, de boutons, d'un herpès, d'escarres, d'irritations cutanées, de coupures, d'un psoriasis, d'un endommagement de la peau dû au soleil, d'un eczéma, d'irritations nasales, d'éruptions cutanées, de réactions allergiques, d'inflammations, d'infections bactériennes, de troubles gastro-intestinaux, d'une dermatite, d'un traitement diurétique, d'une prolifération cutanée ou d'une maladie de la peau.

10. Composition pour le traitement cosmétique d'un humain ou autre animal, comprenant un extrait hydro-alcoolique de la plante *Centipeda cunninghamii,* conjointement avec un véhicule et/ou diluant acceptable, dans laquelle l'extrait hydro-alcoolique est un extrait de matière de plante avec de l'éthanol aqueux dans la gamme de 10 % éthanol/90 % eau à 95 % éthanol/5 % eau.

11. Composition selon la revendication 10, dans laquelle l'extrait hydro-alcoolique est un extrait dans 30 % éthanol/70 % eau.

12. Composition selon la revendication 10 ou 11, dans laquelle l'extrait est préparé à partir d'une partie ou la totalité de la plante.

13. Composition selon l'une quelconque des revendications 10 à 12, dans laquelle l'extrait est préparé par macération, percolation, infusion, décoction, infusion d'huile ou extraction dans alcool/éthanol.

14. Composition selon l'une quelconque des revendications 10 à 13, dans laquelle l'extrait est préparé à partir d'une matière de plante séchée.

15. Composition selon la revendication 10, dans laquelle le véhicule et/ou diluant est un véhicule et/ou diluant oralement ou localement acceptable.

16. Composition selon la revendication 10, dans laquelle le véhicule et/ou diluant comprend un ou plusieurs parmi :
les émulsifiants, les agents protégeant la peau, les agents conditionnant la peau, les humectants, les conservateurs, les solvants, les substances actives d'herbes, les huiles essentielles, les agents de suspension, les agents émulsifiants anioniques, les astringents, les antiseptiques, les agents cicatrisants, les germicides, les anti-inflammatoires, les lotions rafraîchissantes pour la peau, les agents mouillants, les antioxydants, les lubrifiants pour la peau, les émollients, les agents absorbants, les conservateurs, les agents conditionnants pour peau/cheveux, les abrasifs, les détergents, les liants et/ou les agents désodorisants, les solvants, les milieux de dispersion, les charges, les véhicules solides, les solutions aqueuses, les revêtements, les agents antibactériens et antifongiques, les agents isotoniques ou retardant l'absorption.

17. Composition selon l'une quelconque des revendications 10 à 16, sous la forme d'un comprimé ingérable, d'un comprimé oral, d'une capsule, d'une pastille, d'un cachet, d'une plaquette, d'une suspension dans un liquide aqueux, d'un sirop, d'un élixir, d'une émulsion, d'une lotion buccale, d'un gel buccal ou d'un bain de bouche, d'une préparation liquide ou semi-liquide, d'une crème, d'une émulsion homogène visqueuse ou semi-solide, d'un gel, d'un liniment, d'une lotion, d'une pommade, d'une peinture, d'une pâte, d'une solution, d'une pulvérisation, d'un bâton ou d'une suspension, d'un déodorant, d'un nettoyant pour le visage, d'une crème ou d'un gel à raser, d'une crème ou d'un gel pour les yeux/le visage, d'un démaquillant, d'un tonifiant, d'un nettoyant, d'un shampoing, d'un conditionneur ou d'un rafraîchisseur d'haleine.
